# EUROPEAN PATENT APPLICATION

(11) **EP 4 497 330 A1**
(43) Date of publication of application: **29.01.2025**
(21) Application number: 24188628.2
(22) Date of filing: 15.07.2024
(51) Int. Cl.: A23G 4/06, A23G 4/10, A23G 4/18, A61B 5/22

(54) **CHEWING FUNCTION ASSESSMENT MATERIAL, CHEWING FUNCTION ASSESSMENT METHOD, AND CHEWING FUNCTION ASSESSMENT KIT**

(30) Priority: 24.07.2023 JP 2023119925
(71) Applicant: GC Corporation, Sunto-gun, Shizuoka 410-1307 (JP)
(72) Inventor: KAKINUMA, Hiroaki, Tokyo, 174-8585 (JP); YOSHINAGA, Masatoshi, Tokyo, 174-8585 (JP); FUKUSHIMA, Syouichi, Tokyo, 174-8585 (JP)
(74) Representative: Müller-Boré & Partner Patentanwälte PartG mbB

(57) **Abstract**

A chewing function assessment material with which it is possible to assess both of a chewing/grinding function and a bolus-of-food forming function simultaneously is provided. The chewing function assessment material contains a gum base and a sugar component. The content of the sugar component is 6% by mass or greater and 50% by mass or less relative to the whole amount of the chewing function assessment material.

## Description

### BACKGROUND OF THE INVENTION

### Field of the Invention

The present disclosure relates to a chewing function assessment material, a chewing function assessment method, and a chewing function assessment kit.

### Description of the Related Art

A process of masticating a food to form a ground product, and a process of mixing the ground product with the saliva to form a bolus of food are referred to generally as chewing. In recent years, an assessment material and an assessment method that can assess the chewing ability have been proposed with a view to, for example, understanding of the health condition, and the like.

For example, in order to assess the chewing efficiency, which is generally referred to as the chewing/grinding function, a non-sugar gummi candy for chewing ability testing, containing a sugar alcohol, polydextrose, gelatin, and esterified carboxyl group-containing polysaccharide at a specific ratio, has been proposed (for example, see Japanese Patent Application Laid-Open Publication No. 2012-170424). In order to assess the bolus-of-food forming function, a method including use of a gum that changes colors depending on the chewing degree, to determine the chewing ability from the color expressed by the gum after being chewed has been proposed (for example, see Japanese Patent Application Laid-Open Publication No. 2013-090830).

### SUMMARY OF THE INVENTION

However, with the invention described in Japanese Patent Application Laid-Open Publication No. 2012-170424, it is difficult to assess the bolus-of-food forming function, because no bolus of food is formed from a ground product resulting from chewing the gummi candy of the invention. With the invention described in Japanese Patent Application Laid-Open Publication No. 2013-090830, it is difficult to assess the chewing/grinding function, because the gum of the invention is an assessment material using a gum base.

With existing chewing function assessment materials including the inventions described in Japanese Patent Application Laid-Open Publication No. 2012-170424 and Japanese Patent Application Laid-Open Publication No. 2013-090830, it is impossible to assess both of the chewing/grinding function and the bolus-of-food forming function simultaneously. A concern about the existing techniques is that the assessment step is complicated, because it is necessary to use an assessment system using different assessment materials for assessing the chewing/grinding function and the bolus-of-food forming function.

Hence, an object of the present disclosure is to provide a chewing function assessment material with which it is possible to assess both of the chewing/grinding function and the bolus-of-food forming function simultaneously.

A chewing function assessment material according to the present disclosure is a chewing function assessment material containing a gum base and a sugar component. A content of the sugar component is 6% by mass or greater and 50% by mass or less relative to a whole amount of the chewing function assessment material.

According to the present disclosure, it is possible to provide a chewing function assessment material with which it is possible to assess both of the chewing/grinding function and the bolus-of-food forming function simultaneously.

### BRIEF DESCRIPTION OF THE DRAWINGS

FIG. 1A is an image illustrating an example of a chewing function assessment material having a tablet shape;
FIG. 1B is an image illustrating an example of a chewing function assessment material having a ground shape (part 1);
FIG. 1C is an image illustrating an example of a chewing function assessment material having a ground shape (part 2);
FIG. 1D is an image illustrating an example of a chewing function assessment material having a bolus-of-food shape; and
FIG. 2 is a table illustrating the results of appearance assessment and glucose elution concentration measurement in Examples.

### DESCRIPTION OF THE EMBODIMENTS

The present disclosure will be described in detail below.

### (Chewing function assessment material)

A chewing function assessment material according to the present disclosure contains a gum base and a sugar component, and may contain other components as needed.

As will be described in detail below, with the chewing function assessment material according to the present disclosure, it is possible to assess both of a chewing/grinding function and a bolus-of-food forming function simultaneously based on the average thickness and the average consistency of a chewed product resulting from chewing the chewing function assessment material.

### <Gum base>

The gum base is mainly blended in order for a bolus of food to be formed, i.e., in order for a ground product to be changed to a gum paste.

The gum base contains a synthetic rubber, and may further contain other components as needed.

### -Synthetic rubber-

The synthetic rubber is not particularly limited and may be appropriately selected in accordance with the intended purpose. Examples of the synthetic rubber include polyisobutylene, polyisoprene, isobutylene-isoprene copolymers, styrene-butadiene rubbers, and the like. These synthetic rubbers may be used alone or in combination of two or more.

### -Other components-

The other components contained in the gum base are not particularly limited and may be appropriately selected in accordance with the intended purpose. Examples of the other components include polyvinyl acetate, gum ester, a wax, an oil or a fat, an emulsifier, a filler, and the like. These may be used alone or in combination of two or more.

A coating layer may be formed on the surface of the gum base. The material used as the coating layer is not particularly limited and may be appropriately selected in accordance with the intended purpose. Examples of the material include sugars (a liquid sugar, a honey, molasses, a starch syrup, powdered sugars, a sugar alcohol, and the like), an antioxidant, food additives, and the like. One of these may be used alone or in combination of two or more.

In a case where the coating layer contains a sugar, it is assumed that the content of the "sugar" in the coating layer is not included in the content of the "sugar component" of the chewing function assessment material.

The content of the gum base is preferably 60% by mass or greater and 80% by mass or less relative to the whole amount of the chewing function assessment material.

When the content of the gum base is 60% by mass or greater relative to the whole amount of the chewing function assessment material, there is an advantage that the chewing function assessment material has a favorable hardness and is easy to chew.

When the content of the gum base is 80% by mass or less relative to the whole amount of the chewing function assessment material, there is an advantage that the chewing function assessment material has a favorable hardness and it is easy to discern a shape change of the chewing function assessment material from a ground shape to a bolus-of-food shape in a chewing process.

The particle diameter of the gum base is not particularly limited and may be appropriately selected in accordance with the intended purpose, yet a particle diameter (D90) of the gum base is preferably 1 mm or less.

The method for measuring the particle diameter of the gum base is not particularly limited. For example, it is possible to measure the particle diameter of the gum base by using a particle size distribution measuring instrument (laser diffraction/scattering type).

The amount of 20 mesh-on particles in the gum base is not particularly limited, yet is preferably 10% by mass or less relative to the whole amount of the gum base because the chewing function assessment material can have a favorable hardness.

As the gum base, an appropriately synthesized product may be used or a commercially available product may be used. Examples of the commercially available product of the gum base include product name: HEALTH IN GUM PWD-04 (blend powder gum base, D90 = 0.95 mm, an amount of 20 mesh-on particles = 4% by mass relative to the whole amount, available from CAFOSA GUM S.A.), and the like.

### <Sugar component>

The sugar component is mainly blended for tablet formation, and control of the hardness of the chewing function assessment material.

The sugar component is not particularly limited and may be appropriately selected in accordance with the intended purpose. Examples of the sugar component include: monosaccharides such as arabinose, galactose, xylose, glucose, fructose, and the like; disaccharides such as isotrehalose, sucrose, trehalulose, trehalose, neotrehalose, palatinose (isomaltulose), maltose, lactose, and the like; oligosaccharides such as soybean oligosaccharide, invert sugars, starch syrups, and the like; and sugar alcohols such as xylitol, glycerol, sorbitol, maltitol, mannitol, reduced palatinose, reduced maltose starch syrups, reduced starch syrups, and the like. Among these sugar components, anhydrous glucose and reduced palatinose are preferable.

In the present disclosure, the content of the sugar component is 6% by mass or greater and 50% by mass or less and preferably 10% by mass or greater and 40% by mass or less relative to the whole amount of the chewing function assessment material.

When the content of the sugar component is 6% by mass or greater relative to the whole amount of the chewing function assessment material, there is an advantage that the chewing function assessment material has a favorable hardness and it is easy to discern a shape change of the chewing function assessment material from a ground shape to a bolus-of-food shape in a chewing process.

When the content of the sugar component is 50% by mass or less relative to the whole amount of the chewing function assessment material, there is an advantage that the chewing function assessment material has a favorable hardness and is easy to chew.

### <Other components>

The chewing function assessment material according to the present disclosure may contain other components in addition to the components described above.

The other components are not particularly limited and may be appropriately selected in accordance with the intended purpose. Examples of the other components include additives, a colorant, and the like. Specific examples of the additives include a dispersant, a humectant, a stabilizer, an anti-adhesive agent for tablets, a bubble stabilizer, a binder, and the like.

Examples of the dispersant, the humectant, and the stabilizer include sucrose fatty acid ester.

Examples of the stabilizer and the anti-adhesive agent include powder cellulose.

Examples of the bubble stabilizer and the binder include hydroxypropyl cellulose.

The shape of the chewing function assessment material is not particularly limited, yet a tablet form is preferable. Examples of the tablet shape include a disk shape, an annular shape, a plate shape, a star shape, a spherical shape, a bar shape, and the like. It is preferable that the chewing function assessment material undergoes shape change as the chewing time passes. More specifically, it is preferable that the chewing function assessment material has a ground shape in an initial period of chewing, and has a bolus-of-food shape in a later period of chewing.

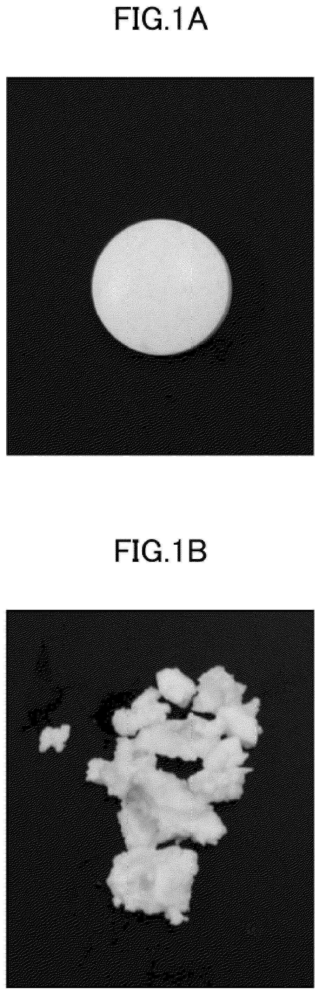

Shape changes of the chewing function assessment material will be described in detail with reference to the FIGS. 1A to 1D.

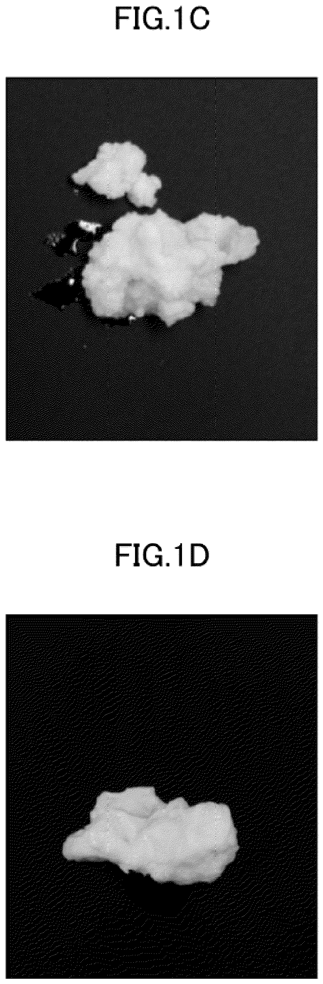

FIG. 1A is an image illustrating an example of the chewing function assessment material having a tablet shape. FIGS. 1B and 1C are images illustrating an example of the chewing function assessment material having a ground shape. FIG. 1D is an image illustrating an example of the chewing function assessment material having a bolus-of-food shape.

It is preferable that the chewing function assessment material has a tablet shape before being chewed, as illustrated in FIG. 1A.

It is preferable that the shape of the chewing function assessment material in an initial period of chewing is a ground shape as illustrated in FIGS. 1B and 1C. Here, the "initial period of chewing" means a period from immediately after starting to chew the chewing function assessment material until before a bolus of food is formed. The "ground shape" means a nonuniform shape in which a ground product and an unground product are mixed.

It is preferable that the shape of the chewing function assessment material in a later period of chewing is a bolus-of-food shape as illustrated in FIG. 1D. Here, the "later period of chewing" means a period after a bolus of food is formed as a result of chewing the chewing function assessment material. The "bolus-of-food shape" means a uniform shape in which no unground product exists.

The hardness of the chewing function assessment material is not particularly limited, may be appropriately selected in accordance with the intended purpose, and is preferably 100 N or greater and 180 N or less and more preferably 120 N or greater and 170 N or less.

When the hardness of the chewing function assessment material is 100 N or greater, there is an advantage that tableting compression is facilitated.

When the hardness of the chewing function assessment material is 180 N or less, there is an advantage that the chewing function assessment material is easy to chew and grind.

The method for measuring the hardness of the chewing function assessment material is not particularly limited. An example of the method is as follows.

### [Example of the method for measuring the hardness of the chewing function assessment material]

A compressive fracture test is performed using a precision universal tester Autograph AG-IS (available from Shimadzu Corporation) at a crosshead speed of 1 mm/min, and the average of compressive fracture loads is used as the hardness.

The volume of the chewing function assessment material is not particularly limited, may be appropriately selected in accordance with the intended purpose, yet is preferably 300 mm³ or greater and 2,300 mm³ or less in terms of facilitating chewing and grinding.

The method for producing the chewing function assessment material is not particularly limited, and an example of the method is as follows.

### [Example of the method for producing the chewing function assessment material]

A mixture (1,200 mg) of the materials of the chewing function assessment material is tableted at a tableting pressure of 1.0 t, to produce a chewing function assessment material having a disk shape having a diameter of 15 mm and a thickness of 7 mm.

The method for verifying the gum base and the sugar component in the chewing function assessment material is not particularly limited and may be appropriately selected in accordance with the intended purpose. For example, the following methods may be employed.

### [Example of an analyzing method for identifying the gum base and the sugar component]

The blended components can be identified based on infrared absorption spectrums of the assessment material that are obtained by a total reflection measuring method of Fourier transform infrared spectroscopy.

### [Example of an analyzing method for quantifying the contents of the blended components]

The assessment material is dissolved in an organic solvent or water, to extract the gum base or the sugar component, to prepare a sample liquid. By the high-performance liquid chromatography method, the molecular species of the sample liquid are identified based on the detection positions in the chromatogram. The concentration of each component can be calculated based on the peak area.

### (Chewing function assessment method)

In a chewing function assessment method according to the present disclosure, a chewing function assessment material is chewed for a certain period of time to form a chewed product, and the chewing function is assessed based on the properties or the appearance of the chewed product.

Here, the "properties" are not particularly limited so long as a chewing function, i.e., a chewing/grinding function and a bolus-of-food forming function can be assessed. Examples of the properties include the average thickness, the average consistency, and the like of the chewed product. More specifically, the chewing/grinding function can be assessed based on the average thickness of the chewed product. The bolus-of-food forming function can be assessed based on the average consistency of the chewed product.

Moreover, the chewing function assessment method according to the present disclosure can sensorily assess the chewing/grinding function and the bolus-of-food forming function based on the appearance (e.g., a captured image, a developed color, or the like) of the chewed product.

The "chewing function assessment material" mentioned above is the same as the one that is described under the foregoing item (Chewing function assessment material). Therefore, overlapping descriptions will be omitted.

The "certain period of time" is not particularly limited and may be appropriately set in accordance with the composition of the chewing function assessment material, the health condition of the test subject, and the like. Yet, the certain period of time is preferably 10 seconds or longer and 40 seconds or shorter in terms of facilitating assessment of the chewed product.

The method for measuring the average thickness of the chewed product is not particularly limited. For example, the average thickness can be measured by the following method.

### [Example of the method for measuring the average thickness of a chewed product]

The average thickness of the chewed product can be measured based partly on Japanese Industrial Standards (JIS) T 6513:2019, Dental Elastomeric Impression Materials, 7.2-Consistency test. Specifically, a chewed product resulting from the certain period of time of chewing is put in the center of a glass plate covered with a polyethylene sheet, a polyethylene sheet and a glass plate are placed on them in this order, and a load of 14 N is immediately applied. The load is removed at 5 seconds after the start of loading, and after 15 minutes or longer has passed, the thickness of the chewed product compressed under the load is measured using a caliper. The number of samples is three or four, and the average of the samples is used as the average thickness of the chewed product.

The method for measuring the average consistency of the chewed product is not particularly limited. For example, the average consistency can be measured by the following method.

### [Example of the method for measuring the average consistency of a chewed product]

The average consistency can be measured based partly on JIS T 6513:2019, Dental Elastomeric Impression Materials, 7.2-Consistency test. Specifically, a chewed product resulting from being chewed for a certain period of time is put in the center of a glass plate covered with a polyethylene sheet, another polyethylene sheet and another glass plate are placed on them in this order, and a load of 14 N is immediately applied. The load is removed at 5 seconds after the start of loading, and the chewed product is left to stand still for 15 minutes or longer. The maximum and minimum dimensions between parallel cutting lines of the chewed product after being left to stand still are measured using a caliper, and the average of the dimensions is calculated. The number of samples is three or four, and the average of the samples is used as the average consistency of the chewed product.

It is possible to perform the method for assessing the chewing/grinding function based on the average thickness, and the method for assessing the bolus-of-food forming function based on the average consistency separately. Yet, it is preferable to perform them simultaneously. In other words, the chewing function assessment material according to the present disclosure enables one sample (chewed product) to be assessed in terms of two items, namely the chewing/grinding function based on measured values of the thickness, and the bolus-of-food forming function based on measured values of the consistency.

The chewing function assessment method may include another assessment method in addition to the chewing/grinding function assessment method based on the average thickness and the bolus-of-food forming function assessment method based on the average consistency.

The other assessment method is not particularly limited. An example of the other assessment method is a chewing/grinding function assessment method that is based on a glucose concentration in the saliva after chewing the chewing function assessment material.

The method for measuring the glucose concentration in the saliva is not particularly limited. For example, the glucose concentration may be measured by the following method.

### [Example of the method for measuring the glucose concentration in the saliva]

A chewed product resulting from chewing and water (10 mL) are put in the mouth cavity, and then the chewed product and the water are spat out into a container on which a filtration mesh is placed, to separate the chewed product and the filtrate from each other. The concentration of glucose contained in the filtrate is measured using a glucose amount measuring instrument (e.g., a Glucosensor GS-IIN, available from GC Corporation).

### (Chewing function assessment kit)

A chewing function assessment kit according to the present disclosure includes a chewing function assessment material, and may include other members.

The "chewing function assessment material" mentioned above is the same as the one that is described under the foregoing item (Chewing function assessment material). Therefore, overlapping descriptions will be omitted.

Examples of the other members include a manual of instructions for use, polyethylene sheets, glass plates, and a ruler for measuring the thickness and the consistency of a chewed product, and a glucose measuring instrument and a filtration mesh for measuring the glucose concentration in saliva.

The uses of the chewing function assessment material are not particularly limited. Examples of the uses of the chewing function assessment material include a food, a chewing function training food, and the like.

### Examples

The present disclosure will further be described below by way of Examples. In the following description, values without units, and "part" or "%" are mass-based unless otherwise particularly noted.

### <Panelist selection>

A gummi chewing ability test was performed in the manner described below, to judge the chewing ability of ten panelists.

After a tester gummi (GLUCORUM, obtained from GC Corporation) was chewed on the main chewing side, water (10 mL) was poured into the mouth cavity, the chewed product and the water were spat out into a container on which a filtration mesh was placed, to separate the chewed product and the filtrate from each other. The concentration of glucose eluted into the filtrate was measured using a glucose amount measuring instrument (a Glucosensor GS-IIN, obtained from GC Corporation). The ten panelists were sorted into three groups based on the following judgment criteria.
- Judgment criteria-
- Group A: The glucose concentration was 150 mg/dL or higher (the chewing ability was normal, four panelists)
- Group B: The glucose concentration was 100 mg/dL or higher and lower than 150 mg/dL (the chewing ability was poor, three panelists)
- Group C: The glucose concentration was lower than 100 mg/dL (the chewing ability was extremely poor, three panelists)

### (Example 1)

### <Production of chewing function assessment material>

Materials were mixed based on the composition and blending amounts indicated in Table 1 below, to prepare a mixture of Example 1. The mixture (1,200 mg) was tableted at a tableting pressure of 1.0 t, to produce a chewing function assessment material of Example 1 having a disk shape having a diameter of 15 mm and a thickness of 7 mm.

A compressive fracture test was performed using a precision universal tester Autograph AG-IS (obtained from Shimadzu Corporation) at a crosshead speed of 1 mm/min, and the hardness of the chewing function assessment material of Example 1 was calculated as an average compressive fracture load. The result is indicated in Table 1.

**Table 1**

| | | Ex. | | | Comp. Ex. | |
|---|---|---|---|---|---|---|
| | | 1 | 2 | 3 | 1 | 2 |
| Gum component | Gum base | 60 | 70 | 77 | 85 | 0 |
| Sugar component | Glucose | 10 | 10 | 10 | 0 | 40 |
| | Reduced palatinose | 20 | 10 | 8 | 5 | 50 |
| Additives | Sucrose fatty acid ester | 5 | 5 | 2 | 5 | 5 |
| | Powder cellulose | 3 | 3 | 1 | 3 | 3 |
| | Hydroxypropyl cellulose | 2 | 2 | 2 | 2 | 2 |
| Total | | 100 | 100 | 100 | 100 | 100 |
| Hardness [/N] | | 172 | 165 | 148 | 85 | 280 |

The details of the materials indicated in Table 1 are as follows.
- Gum base: HEALTH IN GUM PWD-04 (blend powder gum base, D90 = 0.95 mm, an amount of 20 mesh-on particles = 4% by mass relative to the whole amount, obtained from CAFOSA GUM S.A.)
- Glucose (obtained from San-ei Sucrochemical Co., Ltd.)
- Reduced palatinose (obtained from Mitsui DM Sugar Co., Ltd.)
- Sucrose fatty acid ester (obtained from Mitsubishi Chemical Group Corporation)
- Powder cellulose (obtained from Nippon Paper Industries Co., Ltd.)
- Hydroxypropyl cellulose (obtained from Nippon Soda Co., Ltd.)

### <Chewing test>

The chewing function assessment material of Example 1 was chewed for a predetermined period of time (10 seconds, 20 seconds, or 30 seconds), and spat out. The average thickness and the average consistency of the spat-out chewed product were assessed as follows. The results are indicated in Tables 2 and 3.

### [Method for measuring the average thickness of the chewed product]

The average thickness was measured based partly on JIS T 6513:2019, Dental Elastomeric Impression Materials, 7.2-Consistency test. Specifically, a chewed product resulting from being chewed for a certain period of time was put in the center of a glass plate covered with a polyethylene sheet, another polyethylene sheet and another glass plate were placed on them in this order, and a load of 14 N was immediately applied. The load was removed at 5 seconds after the start of loading, and after 15 minutes or longer passed, the thickness of the chewed product compressed under the load was measured using a caliper. The number of samples was three or four, and the average of the samples was used as the average thickness.

### [Example of the method for measuring the average consistency of the chewed product]

The average consistency was measured based partly on JIS T 6513:2019, Dental Elastomeric Impression Materials, 7.2-Consistency test. Specifically, the chewed product resulting from the certain period of time of chewing was put in the center of a glass plate covered with a polyethylene sheet, a polyethylene sheet and a glass plate were placed on them in this order, and a load of 14 N was immediately applied. The load was removed at 5 seconds after the start of loading, and the chewed product was left to stand still for 15 minutes or longer. The maximum and minimum dimensions between parallel cutting lines of the chewed product after being left to stand still were measured using a caliper, and the average of the dimensions was calculated. The number of samples was three or four, and the average of the samples was used as the average consistency of the chewed product.

### (Examples 2 and 3, and Comparative Examples 1 and 2)

Assessment was performed in the same manner as in Example 1, except that the composition and the blending amounts were changed as indicated in Table 1. The results are indicated in Tables 2 and 3.

**Table 2**

| | | Ex. 1 | | | Ex. 2 | | | Ex. 3 | | |
|---|---|---|---|---|---|---|---|---|---|---|
| | | A | B | C | A | B | C | A | B | C |
| Average thickness of chewed product [mm] | Chewing time; 10 sec | 4.2 | 6.2 | 6.2 | 3.5 | 5.6 | 5.7 | 3.3 | 5.2 | 5.4 |
| | Chewing time: 20 sec | 3.5 | 4.3 | 4 . 9 | 2.7 | 3.8 | 4.3 | 2.4 | 3.4 | 4.0 |
| | Chewing time: 30 sec | 2.9 | 3.4 | 3.6 | 2.2 | 2.3 | 3.0 | 1.8 | 1.9 | 2.7 |
| Average consistency of chewed product [mm] | Chewing time; 10 sec | 15.9 | 11.4 | 11.9 | 16.5 | 12.0 | 12.4 | 16.9 | 12.4 | 12.7 |
| | Chewing time: 20 sec | 19.8 | 16.8 | 12.8 | 20.3 | 17.3 | 13.4 | 20.7 | 17.7 | 13.8 |
| | Chewing time: 30 sec | 23.2 | 20.0 | 17.4 | 23.7 | 20.5 | 17.8 | 24.2 | 21.0 | 18.3 |

**[Table 3]**

| | | Comp. Ex. 1 | | | Comp. Ex. 2 | | |
|---|---|---|---|---|---|---|---|
| | | A | B | C | A | B | C |
| Average thickness of chewed product [mm] | Chewing time; 10 sec | 1.8 | 1.9 | 2.0 | 6.4 | 8.7 | 9.5 |
| | Chewing time: 20 sec | 1.7 | 1.7 | 1.7 | Dissolved | Dissolved | 8.7 |
| | Chewing time: 30 sec | 1.7 | 1.7 | 1.7 | Dissolved | Dissolved | 7.6 |
| Average consistency of chewed product [mm] | Chewing time; 10 sec | 28.0 | 27.3 | 26.7 | Unmeasurable | Unmeasurable | Unmeasurable |
| | Chewing time: 20 sec | 28.4 | 28.2 | 27.7 | Dissolved | Unmeasurable | Unmeasurable |
| | Chewing time: 30 sec | 28.7 | 28.0 | 27.3 | Dissolved | Dissolved | Dissolved |

### <Glucose concentration measurement>

In the group B, a chewed product of Example 2 and water (10 mL) were put in the mouth cavity of each panelist, and then the chewed product and the water were spat out into a container on which a filtration mesh was placed, to separate the chewed product and the filtrate from each other.

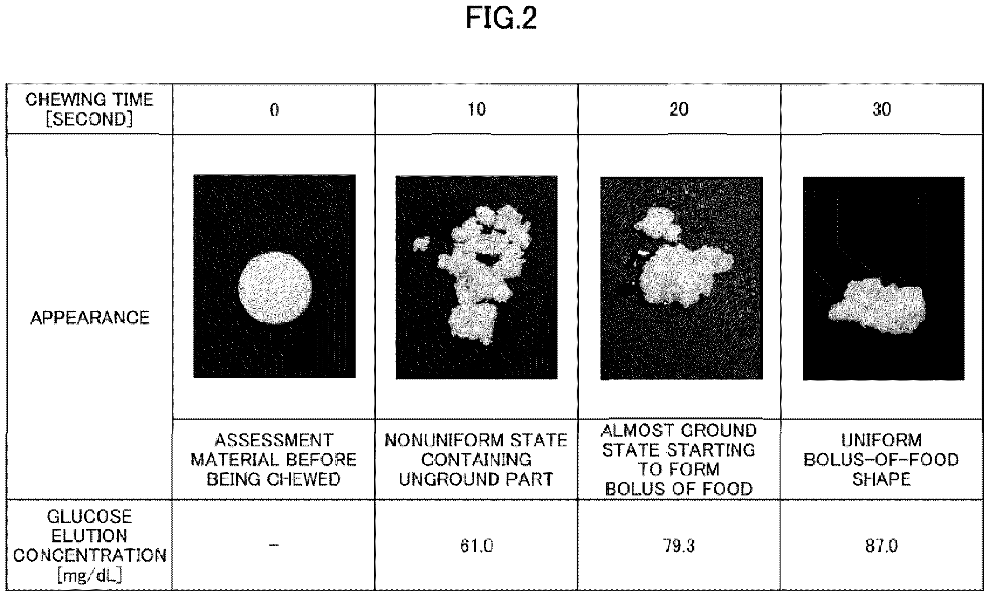

The concentration of glucose contained in the filtrate was measured using a glucose measuring instrument (a Glucosensor GS-IIN, obtained from GC Corporation). The results and the appearances are indicated or illustrated in FIG. 2.

In each of Examples 1 to 3, the chewed products had variations in the average thickness and the average consistency depending on the panelists' chewing abilities. That is, by comparing the chewed products based on the average thickness having correlation with the sugar component grinding degree, it was possible to assess the chewing/grinding abilities. Similarly, by comparing the chewed products based on the average consistency, it was possible to assess the bolus-of-food forming abilities.

In Comparative Example 1, chewed products having a constant thickness and a constant consistency were obtained irrespective of the panelists' chewing abilities. Therefore, it was difficult to assess the chewing/grinding function and the bolus-of-food forming function.

In Comparative Example 2, although it was found that the chewed products had variation in the average thickness depending on the panelists' chewing abilities, it was difficult to assess the chewing/grinding function because the sugar component of the chewed products mixed with and dissolved in the saliva, making it impossible to sufficiently confirm shape change of the chewed products. Moreover, because no bolus of food was obtained, it was also difficult to assess the bolus-of-food forming function.

With the chewing function assessment material according to the present disclosure, it is possible to assess the chewing/grinding function and the bolus-of-food forming function simultaneously, easily, and at low costs, by performing comparison with normal chewed product properties (i.e., the average thickness and the average consistency of a chewed product of a panelist who has the normal chewing ability). Moreover, it is possible to assess the chewing/grinding function in greater detail by performing glucose concentration measurement in combination.

Aspects of the present disclosure are, for example, as follows.
<1> A chewing function assessment material, including:
   a gum base; and
   a sugar component,
   wherein a content of the sugar component is 6% by mass or greater and 50% by mass or less relative to a whole amount of the chewing function assessment material.
<2> The chewing function assessment material according to <1>,
   wherein the chewing function assessment material has a tablet shape.
<3> The chewing function assessment material according to <2>,
   wherein the chewing function assessment material undergoes shape change as chewing time passes, and
   the chewing function assessment material has a ground shape in an initial period of chewing, and has a bolus-of-food shape in a later period of chewing.
<4> The chewing function assessment material according to any one of <1> to <3>,
   wherein a content of the gum base is 60% by mass or greater and 80% by mass or less relative to a whole amount of the chewing function assessment material.
<5> The chewing function assessment material according to any one of <1> to <4>,
   wherein the sugar component contains either or both of anhydrous glucose and reduced palatinose, and
   the content of the sugar component is 10% by mass or greater and 40% by mass or less relative to the whole amount of the chewing function assessment material.
<6> The chewing function assessment material according to any one of <1> to <5>,
   wherein the chewing function assessment material has a volume of 300 mm³ or greater and 2,300 mm³ or less.
<7> A chewing function assessment kit, including:
   the chewing function assessment material of any one of <1> to <6>.
<8> A chewing function assessment method, including:
   chewing a chewing function assessment material for a certain period of time, to form a chewed product, the chewing function assessment material containing a gum base and a sugar component, wherein a content of the sugar component is 6% by mass or greater and 50% by mass or less relative to a whole amount of the chewing function assessment material; and
   assessing a chewing function based on a property or appearance of the chewed product.
<9> The chewing function assessment method according to <8>,
   wherein a chewing/grinding function is assessed based on an average thickness of the chewed product.
<10> The chewing function assessment method according to <8> or <9>,
   wherein a bolus-of-food forming function is assessed based on an average consistency of the chewed product.
<11> The chewing function assessment method according to any one of <8> to <10>,
   wherein a chewing/grinding function is assessed based on a glucose concentration in saliva after the chewing function assessment material is chewed.

The chewing function assessment material according to any one of <1> to <6>, the chewing function assessment kit according to <7>, and the chewing function assessment method according to any one of <8> to <11> can solve the various problems in the related art and achieve the object of the present disclosure.

## Claims

1. A chewing function assessment material, comprising:
a gum base; and
a sugar component,
wherein a content of the sugar component is 6% by mass or greater and 50% by mass or less relative to a whole amount of the chewing function assessment material.

2. The chewing function assessment material according to claim 1,
wherein the chewing function assessment material has a tablet shape.

3. The chewing function assessment material according to claim 2,
wherein the chewing function assessment material undergoes shape change as chewing time passes, and
the chewing function assessment material has a ground shape in an initial period of chewing, and has a bolus-of-food shape in a later period of chewing.

4. The chewing function assessment material according to claim 1,
wherein a content of the gum base is 60% by mass or greater and 80% by mass or less relative to the whole amount of the chewing function assessment material.

5. The chewing function assessment material according to claim 1,
wherein the sugar component contains either or both of anhydrous glucose and reduced palatinose, and
the content of the sugar component is 10% by mass or greater and 40% by mass or less relative to the whole amount of the chewing function assessment material.

6. The chewing function assessment material according to claim 1,
wherein the chewing function assessment material has a volume of 300 mm³ or greater and 2,300 mm³ or less.

7. A chewing function assessment kit, comprising:
the chewing function assessment material of any one of claims 1 to 6.

8. A chewing function assessment method, comprising:
chewing a chewing function assessment material for a certain period of time, to form a chewed product, the chewing function assessment material containing a gum base and a sugar component, wherein a content of the sugar component is 6% by mass or greater and 50% by mass or less relative to a whole amount of the chewing function assessment material; and
assessing a chewing function based on a property or appearance of the chewed product.

9. The chewing function assessment method according to claim 8,
wherein a chewing/grinding function is assessed based on an average thickness of the chewed product.

10. The chewing function assessment method according to claim 8 or 9,
wherein a bolus-of-food forming function is assessed based on an average consistency of the chewed product.

11. The chewing function assessment method according to claim 8 or 9,
wherein a chewing/grinding function is assessed based on a glucose concentration in saliva after the chewing function assessment material is chewed.
